# EUROPÄISCHE PATENTANMELDUNG

(11) **EP 0 842 634 A1**
(43) Veröffentlichungstag der Anmeldung: **20.05.1998**
(21) Anmeldenummer: 97117915.5
(22) Anmeldetag: 16.10.1997
(51) Int. Cl.: A61B 1/247

(54) **Winkelspiegel**

(30) Priorität: 29.10.1996 DE 29618387 U
(71) Anmelder: Preusse, Peter, Dr., 35041 Marburg (DE)
(72) Erfinder: Preusse, Peter, Dr., 35041 Marburg (DE)
(74) Vertreter: Olbricht, Karl Heinrich, Dipl.-Phys.

(57) **Zusammenfassung**

Ein Winkelspiegel hat einen Spiegelteil (52) mit abgebogener Fassung (20), deren Umlaufrand (25) eine einspringende Lippe (40) aufweist. An dieser ist eine Rastkante (36) eines Spiegelelements (30) im Umlaufrand (25) an oder bei einer Hohlkehle (42) unter der Lippe (40) kraft- und/oder formschlüssig gehalten. Hierzu hat die Lippe (40) bevorzugt einen eckigen Querschnitt mit wenigstens einer Stützflanke (43) für das Spiegelelement (30). Die Deck- und Bodenfläche (32) bzw. (48) des Spiegels (30) kann jeweils von Fasen (34) bzw. (46) begrenzt sein. Die Fassung (20) hat am Innenboden (26) eine Ringnut (24), die einen elastisch-nachgiebigen Silikon-Dichtring (50) aufnimmt, der den Spiegelboden (48) formschlüssig abstützt. Zumindest die Fassung (20) kann ein Kunststoff-Formteil sein. Griffteil (12) und Fassung (20) sind entweder einstückig oder lösbar miteinander verbunden, namentlich verschraubbar.

## Beschreibung

Die vorliegende Erfindung bezieht sich auf einen Winkelspiegel gemäß dem Oberbegriff von Anspruch 1.

Derartige Instrumente werden insbesondere als Mundspiegel für zahnärztliche Behandlungen eingesetzt, aber auch überall dort verwendet, wo auf verhältnismäßig engem Raum "um die Ecke" geblickt werden muß, um Einzelheiten an kleineren Objekten betrachten zu können. Die herkömmlichen Geräte haben einen Griffteil, an den ein Hals und eine abgebogene Fassung anschließen, deren Boden mit einem Umlaufrand versehen ist und ein allgemein rundes Spiegelelement haltert. Nicht selten ist die Anordnung mehrteilig, meist aus Metall, wobei der Spiegel in die Fassung eingeklebt sein kann.

Es ist ein wichtiges Ziel der Erfindung, einen neuartigen Winkelspiegel zu schaffen, der bei einfachem und übersichtlichem Aufbau nicht nur eine kostengünstige Fertigung erlaubt, sondern auch verbesserte Gebrauchseigenschatten besitzt. Die Konstruktion soll namentlich auch erhöhten hygienischen Anforderungen genügen.

Hauptmerkmale der Erfindung sind in Anspruch 1 angegeben. Ausgestaltungen sind Gegenstand der Ansprüche 2 bis 21.

Bei einem Winkelspiegel der Eingangs genannten Art sieht die Erfindung gemäß dem kennzeichnenden Teil von Anspruch 1 vor, daß der Umlaufrand der Fassung eine einspringende Lippe aufweist, hinter der das Spiegelelement verrastbar ist. Diese einfache Befestigung erlaubt es unabhängig von der Ausbildung der übrigen Bauteile, vor allem auch unabhängig von deren Material, den Spiegel mühelos zu montieren und - etwa zu Reinigungs- oder Ersatzzwecken - zu entnehmen.

Dazu trägt es bei, wenn laut Anspruch 2 das Spiegelelement im Umfangsbereich mit wenigstens einer Rastkante versehen ist, welche die Lippe untergreift und nach Anspruch 3 im Umlaufrand der Fassung an oder bei einer Hohlkehle unter der Lippe kraft- und/oder formschlüssig gehalten ist. Dies gewährleistet eine ebenso einfache wie zuverlässige Schnapprast.

Gemäß Anspruch 4 kann die Deckfläche des Spiegelelements von einer oberen Fase begrenzt sein, die bei der Ausbildung von Anspruch 5 in die Rastkante ausläuft. Das ist fertigungstechnisch leicht und wirtschaftlich realisierbar. Vorteilhaft weist die Lippe laut Anspruch 6 einen im wesentlichen runden Querschnitt auf, was das Einsetzen des Spiegelelements erleichtert.

Eine weitere wichtige Ausgestaltung der Erfindung geht aus Anspruch 7 hervor, wonach die Lippe einen im wesentlichen eckigen Querschnitt aufweist. Dies gewährleistet eine besonders einfache und leichte Montage des Spiegelelements in der Spiegelfassung bei gleichzeitig verbesserter Dichtwirkung und Hygiene. Hierbei ist es zweckmäßig wenn die Lippe gemäß Anspruch 8 zwei radial einwärts verlaufende Flanken hat, die einen spitzen Winkel einschließen und in eine bevorzugt scharfkantige Nasenkante auslaufen.

Um Toleranzen in der Glasstärke, im Durchmesser des Spiegelelements und/oder in der Spiegelfassung ausgleichen zu können, sieht Anspruch 9 vor, daß zumindest eine Flanke der Lippe als Stützflanke für das Spiegelelement, namentlich für dessen obere Fase ausgebildet ist, wobei laut Anspruch 10 zwischen der Stützflanke der Lippe und der oberen Fase des Spiegels Flächenschluß besteht.

Laut Anspruch 11 hat die Fassung einen ebenen Innenboden, auf dem eine elastisch-nachgiebige Dichtscheibe und/oder eine selbstklebende Folie aufliegt. Das Spiegelelement wird von dem Boden der Fassung optimal unterstützt und sicher gehalten. Die Dichtscheibe bzw. die Klebefolie sorgen für einen dichten Sitz des Spiegels in der Fassung.

Für die Merkmale von Anspruch 12 wird selbständiger Schutz beansprucht. Danach hat die Fassung an einem Innenboden eine Ringnut, die ein elastisch-nachgiebiges Ringelement aufnimmt. Dies ermöglicht eine wirksame Abstützung und Abpolsterung des Spiegelelements beim Einrasten an bzw. in der Fassung. Die Hygiene-Eigenschaften sind gegenüber Spiegeln herkömmlicher Bauart deutlich verbessert.

Konstruktiv ist es günstig, wenn der Innenrand des Umlaufrandes nach Anspruch 13 eine Begrenzungsfläche der Ringnut bildet. Der darin eingesetzte Dichtring kann seine Dichtwirkung voll entfalten und dichtet das Spiegelelement zusätzlich gegen den Innenrand ab.

Besonders vorteilhaft ist die Gestaltung von Anspruch 14, wonach das Ringelement ein Silikon-Dichtring ist und am Umfangsbereich einer Bodenfläche des Spiegelelements formschlüssig angreift. Sein elastischer Andruck gleicht ebenfalls Toleranzen bei der Spiegelelement-Fertigung aus. Zugleich erzielt man eine Abdichtung des unter dem Spiegel befindlichen Totraums, der dadurch gegen eindringende Flüssigkeiten und mikrobiologische Kontamination geschützt ist. Unabhängig davon ist die Sterilisation jederzeit möglich.

Günstig ist es ferner, wenn laut Anspruch 15 die Bodenfläche des Spiegels von einer unteren Fase begrenzt ist, an der das Ringelement flächig anliegt. Diese einfache Maßnahme gewährleistet sicheren Form- und Dichtschluß.

Die Rastung kann beispielsweise gemäß Anspruch 16 noch dadurch verbessert werden, daß die Fassung am Innenrand eine Umlaufrippe hat, an der eine Umfangs-Riefe des Spiegelelements verrastbar ist. Dadurch erzielt man eine zusätzliche Haltekraft.

Ein Winkelspiegel nach Anspruch 17 zeichnet sich dadurch aus, daß zumindest die Fassung ein Kunststoff-Formteil ist, was nicht nur produktionstechnische Vorteile hat. Die Isolier-Eigenschaften des Materials können bei der Handhabung von Vorteil sein; hinzu kommen Möglichkeiten des Designs, die praktischen Anwendungs-Erfordernissen gerecht werden. Glatte Oberflächen von nicht zu kleinem Krümmungsradius erlauben verletzungsfreie Gleitbewegungen entlang der Mundschleimhaut und sind außerdem mühelos zu reinigen.

Laut Anspruch 18 können Griffteil und Fassung einstückig sein, z.B. in Gestalt eines Kunststoff-Formkörpers, der sich gut anfaßt und bei geringem Gewicht sehr bequem zu handhaben ist.

Alternativ sieht Anspruch 19 vor, daß Griffteil und Fassung miteinander lösbar verbunden sind, beispielsweise nach Anspruch 20 dadurch, daß die Fassung oberhalb einer Biegung einen Gewindeansatz aufweist, der in ein stirnseitiges Gewindeloch am unteren Ende des Griffteils einschraubbar ist. Dazu kann der Gewindeansatz laut Anspruch 21 an einer Schulter oberhalb einer Erweiterung der Fassung angeordnet sein, die konturgleich an den Hals des Griffteils anschließt. Dank dieser Formgebung bleiben die obengeschilderten Vorteile erhalten, während der abschraubbare Spiegelteil separat gehandhabt bzw. gewartet oder ausgetauscht werden kann, sollte der Bedarfsfall eintreten.

Weitere Merkmale, Einzelheiten und Vorteile der Erfindung ergeben sich aus dem Wortlaut der Ansprüche sowie aus der folgenden Beschreibung von Ausführungsbeispielen anhand der Zeichnung. Darin zeigen: 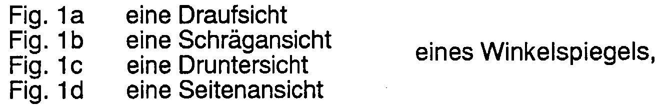
- Fig. 2: eine vergrößerte Schnittansicht entsprechend der Ebene von Linie II-II in Fig. 1d,
- Fig. 3a: ein vergrößerter Ausschnitt einer Schnittansicht eines anderen Ausführungsbeispiels eines Spiegelteils entsprechend der Ebene von Linie II-II in Fig. 1d
- Fig. 3b: eine Schnittansicht des Spiegelteils von Fig. 3a mit einem anderen Spiegelglas, 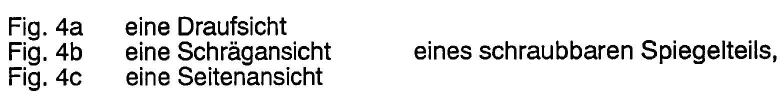
- Fig. 5a: eine Stirnansicht eines Spiegelteils,
- Fig. 5b: eine Ansicht ähnlich Fig. 4a, jedoch von einer abgewandelten Ausführungsform und
- Fig. 6: eine vergrößerte Teil-Schnittansicht ähnlich Fig. 2, jedoch von einer weiteren Bauform.

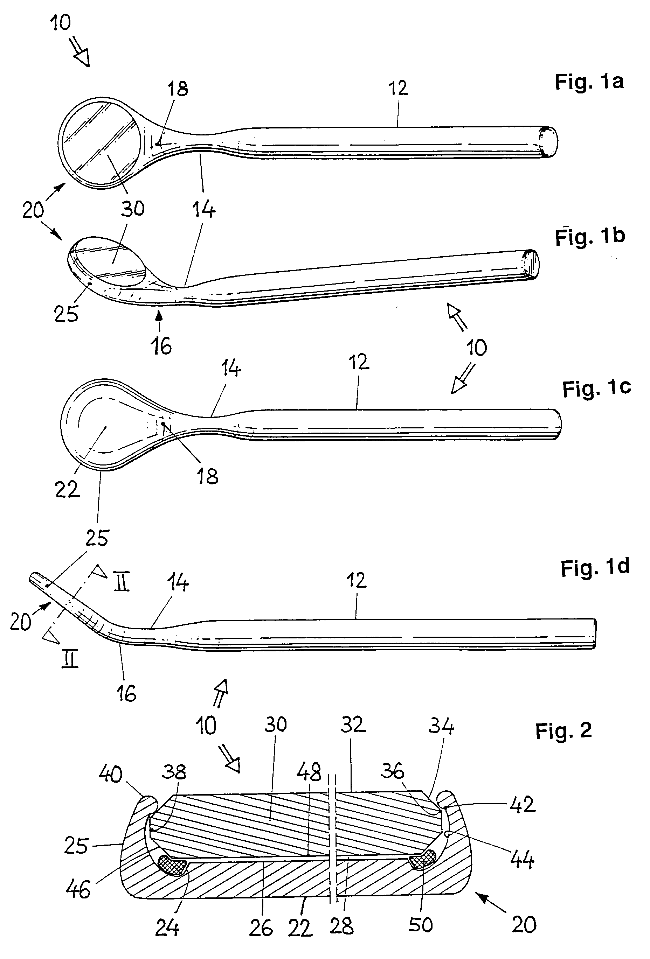

Die verschiedenen Ansichten von Fig. 1a bis 1d zeigen einen allgemein mit 10 bezeichneten Mundspiegel, der einen Griffteil 12 mit einem Hals 14 hat, welcher über eine Biegung 16 und eine Erweiterung 18 in eine Fassung 20 übergeht.

Letztere hat einen Außenboden 22, von dem ein Umlaufrand 25 nach oben absteht, sowie einen Innenboden 26, der umfangsnahe eine Ringnut 24 aufweist (Fig. 2). Die Fassung 20 bildet zusammen mit einem runden Spiegelelement 30 einen Spiegelteil 52.

Der Spiegel 30 ist gewöhnlich eben oder hohl ausgebildet; seine Deckfläche 32 hat eine obere Fase 34. Diese läuft am Umfang 38 in eine Rastkante 36 aus.

Der Umlaufrand 25 der Fassung 20 ist leicht nach außen gewölbt. Er schließt oben mit einer Lippe 40 ab. Diese ist einspringend gestaltet mit einem im wesentlichen runden Querschnitt. Zum Innenrand 44 des Umlaufrandes 25 hin weist die Lippe 40 eine Hohlkehle 42 auf.

Der Innenrand 44 ist konkav gewölbt und läuft nach unten in die Ringnut 24 aus. Diese nimmt einen elastisch-nachgiebigen Dichtring 50 auf, welcher das Spiegelelement 30 an seiner Bodenfläche 48 abstützt, namentlich im Bereich einer unteren Fase 46.

Die Abmessungen der Fassung 20, des Spiegelelements 30 und des Dichtrings 50 sind derart gewählt, daß der Dichtring 50 formschlüssig dichtend an der Fase 46 bzw. an der Bodenfläche 48 des Spiegels 30 anliegt und letzterer beim Eindrücken in die Fassung 20 mit seiner Rastkante 36 hinter der Hohlkehle 42 verrastet. In dieser Raststellung liegt die Lippe 40 des Umlaufrandes 25 auf der oberen Fase 34 des Spiegelelements 30 auf. Dieses ist sicher in der Fassung 20 gehalten.

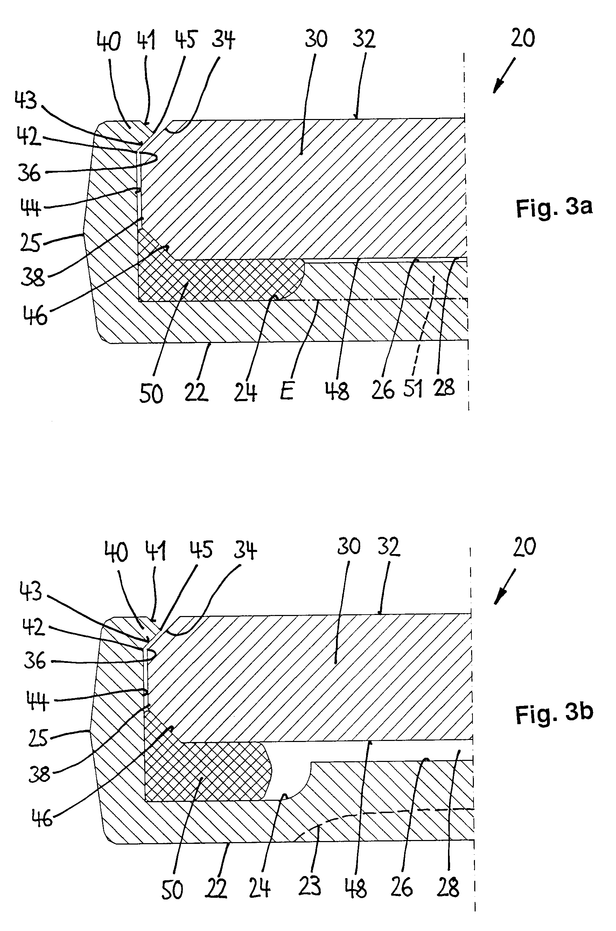

Eine andere Ausführungsform einer Spiegelfassung 20 zeigen Fig. 3a und 3b. Die einspringende Lippe 40 des Umlaufrandes 25 weist einen im wesentlichen eckigen Querschnitt auf. Sie hat eine obere Flanke 41 und eine untere Flanke 43, die einen spitzen Winkel einschließen und in eine nach innen gerichtete scharfkantige Nasenkante 45 auslaufen. Die untere Flanke 43 der Lippe 40 und die obere Fase 34 des Spiegels 30 stehen im gleichen Winkel zu der Deckfläche 32. Gleiches gilt für die obere Flanke 41 der Lippe 40 und die untere Fase 46 des Spiegels 30 in bezug auf die Bodenflächen 26 bzw. 48.

Der Innenrand 44 des Umlaufrandes 25 ist ebenso wie der Umfang 38 des Spiegelelements 30 zylindrisch ausgebildet. Er verläuft senkrecht zum Innenboden 26 der Fassung 20 und bildet im unteren Bereich die äußere Begrenzung der Ringnut 24.

Beim Einsetzen des Spiegelelements 30 in die Fassung 20 gleitet die untere Fase 46 des Spiegelelements 30 flächig an der oberen Flanke 41 der Lippe 40 entlang. Die Fassung 20 wird dadurch etwas erweitert. Sie kann das Spiegelelement 30 vollständig aufnehmen. In Raststellung umgreift der Umlaufrand 25 mit der Lippe 40 das Spiegelelement 30. Dieses sitzt mit seiner Rastkante 36 in der Hohlkehle 42 und stützt sich mit seiner oberen Fase 34 an der unteren Flanke 43 der Lippe 40 flächig ab. Hierdurch ist bereits an dieser Stelle eine gute Dichtwirkung gegeben. Es entstehen keine punktuellen Druckbelastungen des Spiegelelements 30. Der in der Ringnut 24 eingepaßte Dichtring 50 schmiegt sich gleichmäßig dichtend von unten an die untere Fase 46 bzw. an den Boden 48 des Spiegelelements 30 an und drückt das Spiegelelement 30 mit der Fase 34 gleichmäßig gegen die Stützflanke 43 der Lippe 40.

Fig. 3a bzw. 3b zeigen deutlich, daß das Spiegelelement 30 insgesamt formschlüssig und stets dichtend in der Fassung 20 einsitzt. Toleranzen in der Glasstärke und im Durchmesser des Spiegelelements 30 werden - ebenso wie Maßungenauigkeiten der Fassung 20 - durch den Flächenschluß zwischen der Flanke 43 und der Fase 34 sowie durch die Einpassung des Dichtelements 50 in die Ringnut 24 ohne Beeinträchtigung der Dichtwirkung vollständig ausgeglichen. Der Toleranzausgleich im Durchmesser des Spiegelelements 30 erfolgt durch entsprechenden Unterschnitt der Fase 34 unter der Lippe 40 bzw. der Flanke 43. Unterschiedliche Höhen des Spiegelelements 30 werden, wie in Fig. 3b zu sehen, über die Verformung des Dichtring 50 innerhalb der Ringnut 24 ausgeglichen.

Diese ist so gestaltet, daß der Dichtring 50 stets ausreichend Raum zur Ausdehnung besitzt und auch bei dünneren Glasstärken noch eine ausreichend große Fläche des Spiegelelements 30 abdeckt.

Die gesamte Konstruktion gewährleistet nicht nur einen guten und sicheren Halt des Spiegelelements 30 in der Fassung 20. Man erreicht vielmehr eine erheblich verbesserte Dichtwirkung gegenüber herkömmlichen Spiegelkonstruktionen. Verunreinigungen und mikrobiologische Kontaminationen gelangen nicht oder nur sehr schwer in den Totraum 28 zwischen Spiegel 30 und Fassungsboden 26. Die scharfkantig ausgebildete Nasenkante 45 verhindert zudem unmittelbare Schmutzablagerungen an der Verbindungsstelle zwischen Spiegelglas 30 und dem Fassungsrand 41, 45. Hygiene-Versuche haben gezeigt, daß die erfindungsgemäße Spiegelkonstruktion die Hygieneanforderungen der Europäischen Norm EN 29 873 (= ISO 9873) problemlos erfüllt. Der Totraum 28 hinter dem Spiegelelement 30 ist mikrobiologisch nahezu nicht kontaminierbar, was bei angeblich normgemäßen Spiegeln nicht der Fall ist.

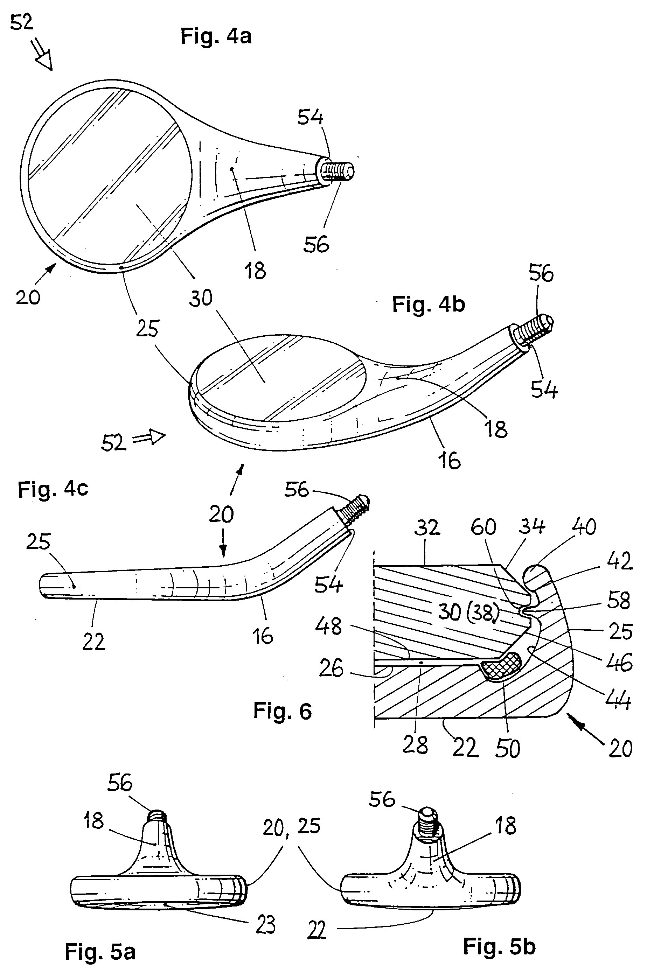

Während der Mundspiegel 10 im Ausführungsbeispiel von Fig. 1a bis 1d insgesamt einstückig ist, zeigen die Fig. 4a bis 4c einen Spiegelteil 52, der mit einem (hier nicht dargestellten) Griffstück verschraubbar ist. Dazu hat der - hier einen ebenen Boden 22 aufweisende - Spiegelteil 52 im Anschluß an die Erweiterung 18 der Fassung 20 eine Schulter 54 mit einem Gewindeansatz 56. Bei im übrigen gleichem Aufbau zeigt Fig. 5a in Stirnansicht einen Spiegelteil 52, dessen Boden 22 konkav gestaltet ist. Im Beispiel der Fig. 5b ist hingegen der Boden 22 nach außen gewölbt.

Die Teil-Schnittansicht von Fig. 6 zeigt eine weitere Variante, bei welcher das Spiegelelement 30 an seinem Umfang 38 eine Riefe 60 aufweist, die mit einer einspringenden Rippe 58 am Innenumfang 44 des Umlaufrandes 25 zusammenwirkt. Dadurch ist eine besonders sichere Halterung des Spiegelelements 30 in der Fassung 20 gewährleistet.

Bevorzugt hat das Spiegelelement 30 eine Dicke von weniger als 2 mm, beispielsweise 1,6 mm. Sein Durchmesser kann zwischen 20 und 25 mm liegen. Ein günstiger Fasen- und/oder Flankenwinkel beträgt 45°. Die Ringstärke des Dichtrings 50 ist bei ausreichender Fassungsbodentiefe auf z.B. 1,2 mm bemeßbar. Die Breite der Ringnut 24 liegt bevorzugt bei 2 mm. Die Shorehärte des Dichtrings 50 beträgt 30 bis 50.

Die Erfindung ist nicht auf die dargestellten Ausführungsformen beschränkt, sondern vielfältig abwandelbar. So kann beispielsweise die Lippe 40 auch andere Querschnittsformen aufweisen, die vergleichbare Rast- und Dichtungseigenschaften gewährleisten.

In einer noch anderen Ausführungsform kann der Dichtring 50 durch eine (in Fig. 3a nur angedeutete) Dichtscheibe 51 ersetzt werden, die den Fassungsboden 26 vollständig überdeckt. Letzterer wäre dann, wie in Fig. 3a durch eine strichpunktierte Linie E angedeutet, ohne Ringnut 24 vollständig eben ausgebildet. Durch die formschlüssige Verbindung zwischen Fassungsboden 26 und Spiegelboden 48 würde ein Totraum 28 gar nicht erst entstehen. Alternativ kann man die Dichtscheibe 51 auch durch eine Klebefolie mit gegebenenfalls elastischen Eigenschaften ersetzen.

Um das Ausbauen des Spiegelelements 30 zu erleichtern, kann man den Fassungsboden 22 zur Mitte hin vertieft ausbilden (siehe Fig. 3b und Fig. 5a). Der Spiegel 30 ließe sich dann durch Eindrücken des Bodens 22 im Bereich der durch gestrichelte Linien angedeuteten Vertiefung 23 einfach und bequem aus der Fassung 20 herausnehmen. Neben einer Rundform können Spiegelelement 30 und Fassung 20 auch eine ovale oder eckige Form aufweisen.

Man erkennt jedoch, daß ein Winkelspiegel nach der Erfindung einen Spiegelteil 52 mit abgebogener Fassung 20 hat, deren Umlaufrand 25 eine einspringende Lippe 40 aufweist, an der eine Rastkante 36 eines Spiegelelements 30 im Umlaufrand 25 an oder bei einer Hohlkehle 42 unter der Lippe 40 kraft- und/oder formschlüssig gehalten ist. Hierzu hat die Lippe 40 bevorzugt einen eckigen Querschnitt mit wenigstens einer Stützflanke 43 für das Spiegelelement 30. Die Deck- und Bodenfläche 32 bzw. 48 des Spiegels 30 kann jeweils von Fasen 34 bzw. 46 begrenzt sein. Die Fassung 20 hat am Innenboden 26 eine Ringnut 24, die einen elastisch-nachgiebigen Silikon-Dichtring 50 aufnimmt, der den Spiegelboden 48 formschlüssig abstützt. Zumindest die Fassung 20 kann ein Kunststoff-Formteil sein. Griffteil 12 und Fassung 20 sind entweder einstückig oder lösbar miteinander verbunden, namentlich verschraubbar.

Sämtliche aus den Ansprüchen, der Beschreibung und der Zeichnung hervorgehenden Merkmale und Vorteile, einschließlich konstruktiver Einzelheiten und räumlicher Anordnungen, können sowohl für sich als auch in den verschiedensten Kombinationen erfindungswesentlich sein.

### Bezugszeichenliste

- E: ebener Boden
- 10: Mundspiegel
- 12: Griff(teil)
- 14: Hals
- 16: Biegung
- 18: Erweiterung
- 20: Fassung
- 22: Außenboden
- 23: Vertiefung
- 24: Ringnut
- 25: Umlaufrand
- 26: Innenboden
- 28: Zwischenraum
- 30: Spiegel(element)
- 32: Deckfläche
- 34: obere Fase
- 36: Rastkante
- 38: Umfang
- 40: Lippe
- 41: Flanke
- 42: Hohlkehle
- 43: Flanke
- 44: Innenrand
- 45: Nasenkante
- 46: untere Fase
- 48: Bodenfläche
- 50: Dichtring
- 51: Dichtscheibe
- 52: Spiegelteil
- 54: Schulter
- 56: Gewindeansatz
- 58: Rippe
- 60: Riefe

## Patentansprüche

1. Winkelspiegel, insbesondere Mundspiegel (10) für zahnärztliche Behandlungen, mit einem Griffteil (12), an den ein Hals (14) und eine abgebogene Fassung (20) anschließen, die einen Boden (22) mit Umlaufrand (25) aufweist und darin ein Spiegelelement (30) haltert, dadurch **gekennzeichnet**, daß der Umlaufrand (25) eine einspringende Lippe (40) aufweist, hinter der das Spiegelelement (30) verrastbar ist.

2. Winkelspiegel nach Anspruch 1, dadurch **gekennzeichnet**, daß das Spiegelelement (30) im Umfangsbereich mit wenigstens einer Rastkante (36) versehen ist, welche die Lippe (40) untergreift.

3. Winkelspiegel nach Anspruch 2, dadurch **gekennzeichnet**, daß die Rastkante (36) im Umlaufrand (25) der Fassung (20) an oder bei einer Hohlkehle (42) unter der Lippe (40) kraft- und/oder formschlüssig gehalten ist.

4. Winkelspiegel nach einem der Ansprüche 1 bis 3, dadurch **gekennzeichnet**, daß die Deckfläche (32) des Spiegelelements (30) von einer oberen Fase (34) begrenzt ist.

5. Winkelspiegel nach den Ansprüchen 2 bis 4, dadurch **gekennzeichnet**, daß die obere Fase (34) in die Rastkante (36) ausläuft.

6. Winkelspiegel nach einem der Ansprüche 1 bis 5, dadurch **gekennzeichnet**, daß die Lippe (40) einen im wesentlichen runden Querschnitt aufweist.

7. Winkelspiegel nach einem der Ansprüche 1 bis 6, dadurch **gekennzeichnet**, daß die Lippe (40) einen im wesentlichen eckigen Querschnitt aufweist.

8. Winkelspiegel nach Anspruch 7, dadurch **gekennzeichnet**, daß die Lippe (40) zwei radial einwärts verlaufende Flanken (41, 43) hat, die einen spitzen Winkel einschließen und in eine bevorzugt scharfkantige Nasenkante (45) auslaufen.

9. Winkelspiegel nach Anspruch 8, dadurch **gekennzeichnet**, daß zumindest eine Flanke (43) der Lippe (40) als Stützflanke für das Spiegelelement (30), namentlich für dessen obere Fase (34) ausgebildet ist.

10. Winkelspiegel nach Anspruch 9, **gekennzeichnet** durch Flächenschluß zwischen der Stützflanke (43) der Lippe (40) und der oberen Fase (34) des Spiegels (30).

11. Winkelspiegel nach einem der Ansprüche 1 bis 10, dadurch **gekennzeichnet**, daß die Fassung (20) einen ebenen Innenboden (26) hat, auf dem eine elastisch-nachgiebige Dichtscheibe (50) und/oder eine selbstklebende Folie aufliegt.

12. Winkelspiegel insbesondere nach einem der Ansprüche 1 bis 11, dadurch **gekennzeichnet**, daß die Fassung (20) an dem Innenboden (26) eine Ringnut (24) hat, die ein elastisch-nachgiebiges Ringelement (50) aufnimmt.

13. Winkelspiegel nach Anspruch 11, dadurch **gekennzeichnet**, daß der Innenrand (44) des Umlaufrandes (25) eine Begrenzungsfläche der Ringnut (24) bildet.

14. Winkelspiegel nach Anspruch 12 oder 13, dadurch **gekennzeichnet**, daß das Ringelement (50) ein Silikon-Dichtring ist und am Umfangsbereich einer Bodenfläche (48) des Spiegelelements (30) formschlüssig angreift.

15. Winkelspiegel nach einem der Ansprüche 12 bis 14, dadurch **gekennzeichnet**, daß die Bodenfläche (48) des Spiegels (30) von einer unteren Fase (46) begrenzt ist, an welcher das Ringelement (50) flächig anliegt.

16. Winkelspiegel nach einem der Ansprüche 1 bis 15, dadurch **gekennzeichnet**, daß die Fassung (20) am Innenrand (44) eine Umlaufrippe (58) hat, an der eine Umfangs-Riefe (60) des Spiegelelements (30) verrastbar ist.

17. Winkelspiegel nach einem der Ansprüche 1 bis 16, dadurch **gekennzeichnet**, daß zumindest die Fassung (20) ein Kunststoff-Formteil ist.

18. Winkelspiegel nach einem der Ansprüche 1 bis 17, dadurch **gekennzeichnet**, daß Griffteil (12) und Fassung (20) einstückig sind.

19. Winkelspiegel nach einem der Ansprüche 1 bis 18, dadurch **gekennzeichnet**, daß Griffteil (12) und Fassung (20) miteinander lösbar verbunden sind.

20. Winkelspiegel nach Anspruch 19, dadurch **gekennzeichnet**, daß die Fassung (20) oberhalb einer Biegung (16) einen Gewindeansatz (56) aufweist, der in ein stirnseitiges Gewindeloch am unteren Ende des Griffteils (12) einschraubbar ist.

21. Winkelspiegel nach Anspruch 20, dadurch **gekennzeichnet**, daß der Gewindeansatz (56) an einer Schulter (54) oberhalb einer Erweiterung (18) der Fassung (20) angeordnet ist, die konturgleich an den Hals (14) des Griffteils (12) anschließt.
